# EUROPÄISCHE PATENTSCHRIFT

(11) **EP 2 364 129 B1**
(45) Veröffentlichungstag und Bekanntmachung des Hinweises auf die Patenterteilung: **19.06.2013**
(21) Anmeldenummer: 09801137.2
(22) Anmeldetag: 04.11.2009
(51) Int. Cl.: A61F 2/58, A61F 2/68

(54) **FINGERELEMENT**
FINGER MEMBER
ELÉMENT DE DOIGT

(30) Priorität: 08.11.2008 DE 102008056520
(43) Veröffentlichungstag der Anmeldung: 14.09.2011
(73) Patentinhaber: Schulz, Stefan, 76149 Karlsruhe (DE)
(72) Erfinder: Schulz, Stefan, 76149 Karlsruhe (DE)
(74) Vertreter: Kerscher, Nicolas Josef
(86) Internationale Anmeldenummer: PCT/DE2009/001537
(87) Internationale Veröffentlichungsnummer: WO 2010/051798

(56) Entgegenhaltungen:
- WO-A1-2007/063266
- WO-A2-2007/076764

## Beschreibung

Die Erfindung betrifft ein Fingerelement gemäß des ersten Patentanspruchs. Es dient als autarkes Element für den Einsatz als künstliche Einzelfingerprothese oder als Komponente einer künstlichen Hand- oder Armprothese.

Es gibt künstliche Fingerelemente als integrale Bestandteile von Hand- oder Armprothesen eingesetzt sind. Sie orientieren sich in ihrer Form und ihrer Beweglichkeit an einen Finger.

Aus der DE 309 367 ist bereits aus der Zeit des ersten Weltkriegs Handprothesen mit mehrgliedrigen gegeneinander wirkenden Daumen- und Zeigefingerelementen bekannt. Die einzelnen Fingergelenke werden über ein Getriebe mit Zahnbogen und Gewindeschnecken positioniert, wobei der Antrieb über Seilzüge und Hebelmechanismen aus der Handfläche heraus erfolgt. Ein motorischer Antrieb ist nicht vorgesehen, ebenso eine individuelle Bewegbarkeit einzelner Finger.

Auch die DE 323 970 von 1919 offenbart einen derartigen Schwenkmechanismus für ein Fingerglied.

Handprothesen mit einem motorischen Schwenkantrieb für zwei Finger gegeneinander offenbaren beispielsweise DE 26 07 499 C3 und US 4.094.016**.** Ein Getriebemotor als Stellelement befindet sich in diesen Konzepten im Handflächenbereich der Prothese.

Die genannten Systeme umfassen jedoch keine autark angetriebene Fingerelemente, wobei alle für den Betrieb erforderlichen Stellelemente im Fingerelement enthalten sind. Die Antriebe sind bei diesen Systemen außerhalb der Fingerelemente angeordnet. Sie eignen sich daher konzeptionell nicht für einen Einsatz als Einzelfingerprothese. Auch ein Einsatz als Komponente in einem Prothesen-Baukastensystem wird hierdurch wesentlich eingeschränkt.

Dagegen offenbaren die DB 698 16 848 T2 sowie DB 198 54 762 C2 je ein Fingerelement mit je einem motorischen Schwenkantrieb mit Gewindeschnecke und Zahnrad pro Gelenk. Die Motoren sind direkt in den Fingergliedern untergebracht.

Bei diesen Fingerelementen ist jedoch das Schneckenrad fest mit der Motorwelle verbunden, sodass bei einer Belastung des Fingerelements hohe Kräfte auf den Motor einwirken können. Ein vorzeitiger Getriebe- oder Motorschaden sowie ein Blockieren des Schneckenantriebes unter Last wird damit begünstigt.

Konstruktionen wie z.B. in DB 319 092 A und US 2005 0021154 A1 beschrieben, bei denen die Bewegung der distalen Fingerglieder über steife praktisch nicht nachgiebige Schub- und Zugstangen erreicht werden soll, wirken meist unrealistisch roboterhaft und eignen sich insbesondere nicht für sensible Greifaufgaben. Auch ist hierbei mit vorzeitigen Überlastungssymptomen zu rechnen.

In der WO 2007/063266, die als nächster Stand der Technik angesehen wird, **A1** wird dagegen ein Prothesenhand mit beweglichen Fingerelementen beschrieben, bei der eine Entkopplung von Motorwelle und Gewindeschnecke über eine dazwischen angeordnete Kegelzahnradübersetzung mit einer Umlenkung der Drehbewegung um etwa einen rechten Winkel realisiert wird.

Mit dem letztgenannten Konzept erhält man zwar eine mechanische Teilentkopplung von Schneckenrad und Motorwelle und damit eine mechanische Entlastung der letztgenannten, allerdings in Verbindung mit zusätzlichen Komponenten und/oder einem größeren Bauvolumen. Letzteres schränkt den Einsatz als Einzelfingerprothese insbesondere für den Ersatz kleinerer Finger aus ästhetischen Gründen wesentlich ein.

Davon ausgehend liegt die Aufgabe der Erfindung darin, ein Fingerelement so zu modifizieren, dass es grundsätzlich als autarkes Element, d.h. auch als Einzelfingerprothese einsetzbar ist. Insbesondere soll das Fingerelement in seiner aktiven und passiven Funktion sowie in seinen Abmessungen einem natürlichen Finger, vor allem auch kleinerer Finger sehr nahe kommen und dabei eine hohe Lebensdauer aufweisen.

Die Aufgabe wird mit einem Fingerelement mit den Merkmalen des Anspruch 1 gelöst. Auf diesen rückbezogene Unteransprüche geben vorteilhafte Ausführungsformen des Fingerelements wieder.

Die Aufgabe wird mit einem Fingerelement mit einer Trägerkomponente, ein an dieses über eine erste Gelenkverbindung gelenkig angebundenes erstes Fingerglied sowie ein an letzteres über eine zweite Gelenkverbindung gelenkig angebundenes zweites Fingerglied gelöst. Ferner ist ein Stellantrieb für die erste Gelenkverbindung vorgesehen. Dieser umfasst einen Motor mit oder ohne eine integrierte Getriebeübersetzung zu einer Antriebswelle. Die Antriebswelle treibt eine Gewindeschnecke eines vorteilhaft selbsthemmenden Schneckengetriebes an, die wiederum in ein Zahnsegment eingreift und dieses synchron der Drehbewegung der Motorwelle bewegt. Zudem umfasst das Fingerelement einen Kopplungsmechanismus zwischen ersten und zweiten Gelenkverbindung.

Das Zahnsegment ist vorzugsweise ein Teil eines Zahnrads oder eines Zahnradsegments um eine Zahnradachse, die vorzugsweise gleichzeitig mit der Schwenkachse der ersten Gelenkverbindung zusammenfällt. Die Erfindung umfasst grundsätzlich auch ein Zahnsegment als Teil einer zahnstange, die beweglich in einem Abstand zur Schwenkachse im Fingerelement angreifend gelagert ist.

Ein wesentliches Merkmal der Erfindung umfasst eine Entkopplung von Motorwelle und Gewindeschnecke in axialer Richtung zur Motorwelle. Die Gewindeschnecke ist vorzugsweise auf die Motorwelle aufgesetzt und ist in Umdrehungsrichtung mit ihr formschlüssig, z.B. über eine Verzahnung oder eine Nutverbindung gekoppelt. Eine axiale Beweglichkeit der Motorwelle in der Gewindeschnecke muss dabei sichergestellt sein.

Als axiale Führung der Gewindeschnecke dient somit nicht der Motor über die Motorwelle, sondern separate Führungen. Sie sind vorzugsweise als Gleitführungen an beiden Stirnseiten der Gewindeschnecke angeordnet.

Es hat sich als vorteilhaft erwiesen, die Führungen und die Gewindeschnecken zu einer Baugruppe zusammenzufassen. Beispielsweise werden die beiden Führungen durch einen starren, bevorzugt einstückigen Rahmen gebildet, in den die Schnecke mit einem geringen Axialspiel eingesetzt ist.

Wird diese Baugruppe als Einheit und/oder das Zahnsegment zudem elastisch nachgiebig in das Fingerelement eingesetzt, entsteht durch diese eine elastische Biegenachgiebigkeit des Fingerelements um die Gelenkverbindung, die einer funktionale Nachbildung eines natürlichen Fingers nahe kommt. Die genannte Baugruppe wird hierzu vorzugsweise in eine elastomere Halterung im Fingerelement eingesetzt, die eine Nachgiebigkeit der Baugruppe in laterale und/oder rotatorische Freiheitsgrade ermöglicht. Das Zahnsegment, das Zahnradsegment oder das Zahnrad ist vorzugsweise frei drehend auf der Schwenkachse gelagert und wird dabei über eine elastomere Elemente elastisch in seiner Drehbewegung gehemmt.

Zur Sicherstellung eines gleich bleibenden Spiels zwischen Gewindeschnecke und Zahnsegment ist vorzugsweise ein Abstandshalter zwischen Schwenkachse und der vorgenannten Baugruppe vorgesehen. Dieser ist vorzugsweise drehbar auf der Schwenkachse gelagert und mit der Drehachse der Gewindeschnecke verbunden. Beispielsweise ist hierzu die Gewindeschnecke in den Führungen nicht nur axial, sondern auch radial gelagert, während der Abstandshalter fix mit den Führungen verbunden ist.

Im Falle einer elastischen Nachgiebigkeit der Baugruppe und damit der Gewindeschnecke relativ zu Motor ist die formschlüssige Lagerung der Gewindeschnecke auf der Motorwelle als eine schwenkbare Drehkupplung vorzusehen, beispielsweise als Gelenkwellenkupplung z.B. mit umlaufenden und in Vertiefungen in der Motorwelle und Innenbohrung der Gewindeschnecke eingreifende Festkörper, z.B. Kugeln eines Kugelrings.

Der Kopplungsmechanismus umfasst vorzugsweise mindestens eine elastische Verbindung z.B. als Zug- und Schubstange zwischen Trägerkomponente und zweitem Fingerglied parallel geschaltet zum ersten Fingerglied. Er besteht weiter bevorzugt aus einer oder zwei Federstabverbindungen (z.B. Federdraht oder Federblech), die exzentrisch zu den Schwenkachsen der ersten und zweiten Gelenkverbindungen an der jeweils benachbarten Trägerkomponente bzw. zweiten Fingerglied angreifen. Für die Nachbildung von Eigenschaften einer natürlichen Hand, d.h. mit einer elastischen Nachgiebigkeit mit vorzugsweise anfänglicher progressiver Federcharakteristik hat sich als vorteilhaft erwiesen, die Drahtverbindungen gebogen oder geknickt zu gestalten. Es entspricht der Mechanik eines natürlichen Fingers, wenn die Zug- und Schubstange in Schließrichtung (z.B. beim Greifen) auf Zug beansprucht ist und damit nicht nur größere Kräfte übertragen kann, sondern auch eine stärkere Progression in der Federcharakteristik aufweist (eine Krümmung oder Knick in einer Federstabverbindung zieht sich bei Zugbelastung gerade und wird damit steifer, im Gegensatz zu einer Belastung unter Druck).

Die Erfindung wird im Folgenden anhand von Ausführungsbeispielen mit den folgenden Figuren näher erläutert. Es zeigen
**Fig.1a** **bis c** jeweils eine seitliche Ansicht einer Ausführungsform in gestreckter (a) sowie in zwei gebeugten (b, c) Stellungen,
**Fig.2** eine prinzipielle Schnittdarstellung der Ausführungsform in gestreckter Stellung,
**Fig.3a** **und b** jeweils eine Ansicht einer Handprothese, ausgestattet mit Fingerelementen sowie
**Fig.4a** **bis d** jeweils eine Ansicht einer Ausführungsform als Daumenelement in verschiedenen Stellungen.

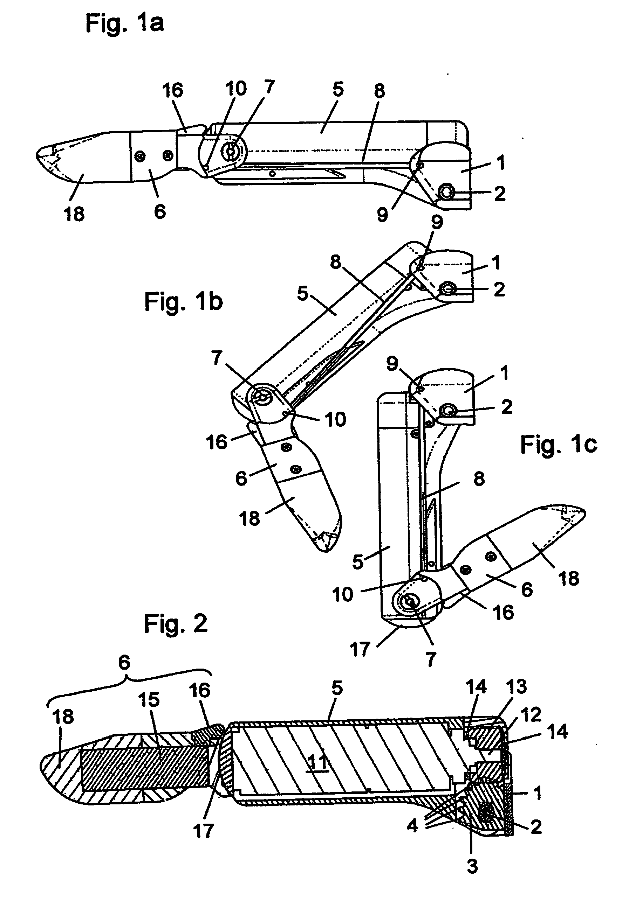

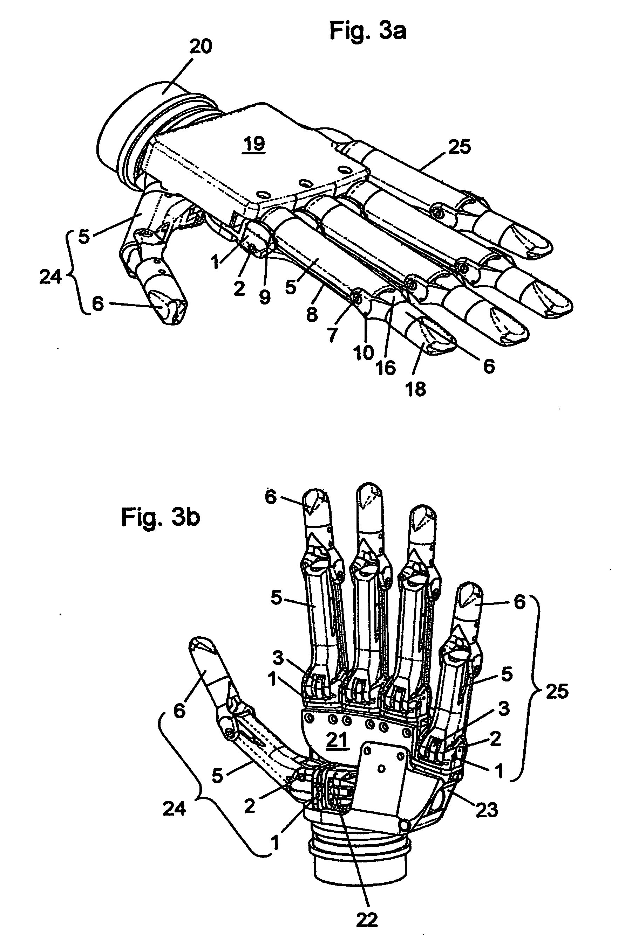

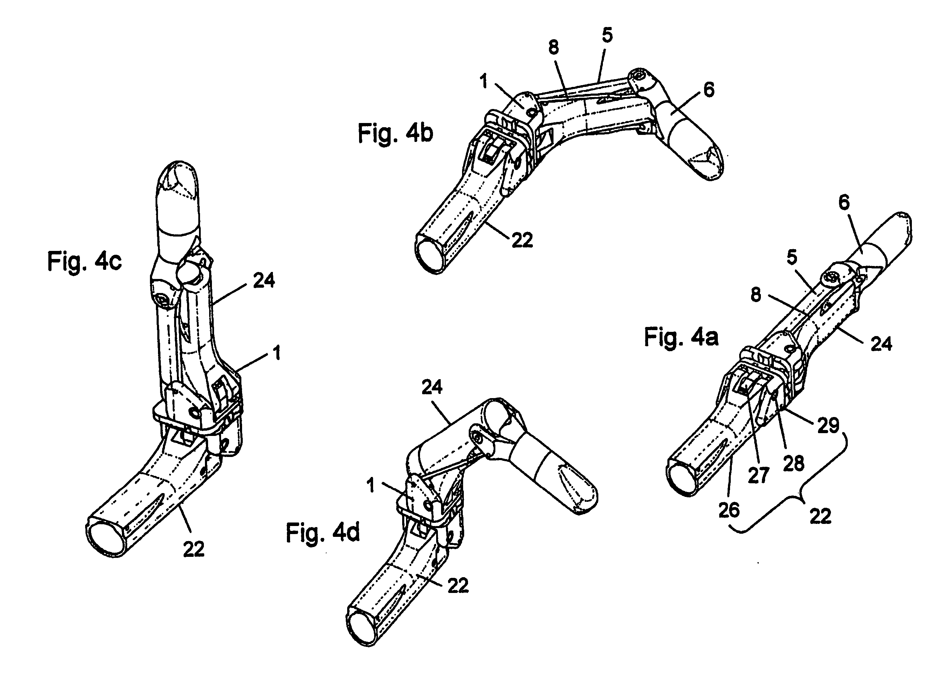

Die dargestellten Ausführungsformen des Fingerelements umfassen eine jeweils eine Trägerkomponente **1** mit einer ersten Gelenkachse **2** und Zahnradsegment **3** mit mehreren Zahnsegmenten **4.** Die Trägerkomponente **1** weist nicht weiter dargestellte Mittel wie Montagebohrungen für die Befestigung des Fingerelements z.B. an eine Handprothese (vgl. **Fig.3a** **und b**) auf. Die erste Gelenkachse **2** dient zugleich als Lagerung des Zahnradsegments und als Schwenkachse für das erste Fingerglied **5.** Das erste Fingerglied ist wiederum über eine zweite Gelenkverbindung mit einem zweiten Fingerglied 6 verbunden, wobei die zweite Gelenkachse **7** die Schwenkachse für das zweite Fingerglied bildet. Die Schwenkbewegung vom erstem und zweiten Fingerglied um die jeweiligen Schwenkachsen sind über einen Kopplungsmechanismus-gekoppelt (vgl. **Fig.1a** **bis c** und **Fig.4a** **bis d**)**.** Dieser umfasst in den Ausführungsbeispielen zwei parallel zueinander beidseitig des ersten Fingerglieds 5 angeordnete elastische Federstabverbindungen **8**, die jeweils an der Trägerkomponente und an dem zweiten Fingerglied drehbar in je einer Lagerbohrung **9** und bzw. **10** exzentrisch zu der ersten bzw. zweiten Gelenkachse **2** bzw. **7** eingesetzt eingreifen.

**Fig.2** zeigt einen Querschnitt durch ein Fingerelement. Ein motorischer Antrieb 11 für eine direkt auf die Antriebswelle **12** aufgesetzte Gewindeschnecke **13** befindet sich im Innern des rohrförmige ersten Fingerglieds **5.** Die Gewindeschnecke ist über eine nicht näher dargestellte Feder-Nut-Verbindung in Umdrehungsrichtung formschlüssig miteinander gekoppelt, axial aber verschiebbar auf der Antriebsachse aufgeschoben. Die Gewindeschnecke ist im Ausführungsbeispiel in radialer Richtung auf der Antriebswelle geführt, wird aber in ihrer axiale Verschiebbarkeit durch zwei Führungen **14** vorzugsweise spielfrei begrenzt. Die Materialien für die Gewindeschnecke weisen zu den Materialien der Führungen vorzugsweise einen geringen Gleitreibungskoeffizienten sowie hohe abrasive Beständigkeiten auf. Beispielsweise ist die Gewindeschnecke wegen der zu erwartenden hohen Belastung des Stellantriebs aus Messing oder Stahl, die Führungen aus einem bevorzugt trocken schmierenden Gleitlagerbuchsenmaterial wie einem PTFE-Material oder einer Gleitlagerbronze gefertigt.

Der Antrieb **11** umfasst zumindest einen elektrischen Motor als Stellelement, optional auch eine Getriebeeinheit und/oder für einen Einsatz z.B. als autarke Fingerprothese eine elektrische Spannungsquelle sowie eine Steuerelektronik (Batterie, Akku etc.), wobei insbesondere die letztgenannten Komponenten sich auch im Kern **15** des zweiten Fingerglieds **6** anordnen lassen.

Das zweite Fingerglied **6** umfasst zudem eine elastische Fingerkuppe **18** vorzugsweise aus einem Elastomer sowie einen auf eine Gleitfläche **17** (vorzugsweise ein einsetzbares Deckelelement z.B. aus Kunststoff) des ersten Fingerglieds **5** wirkenden Abstreifer **16.**

Es hat sich als vorteilhaft erwiesen einen optionalen taktilen Sensor in die Fingerkuppe **18** und/oder in den Kern **15** zu integrieren und/oder Dehnmessstreifen auf eines der Fingerglieder zu befestigen, der bzw. die ein taktiles Signal direkt in die Steuerelektronik (vorzugsweise dann im Kern angeordnet) weiterleitet. Das taktile Signal dient vorzugsweise einer sensiblen Erkennung der angreifenden Kraft und einer taktilen Rückmeldung einer Greifkraft an den Prothesenträger (Kraftrückkopplung) alternativ oder zusätzlich zur Stromaufnahme des motorischen Antriebs und damit der Kraftbegrenzung bei der Bewegungssteuerung.

**Fig.3a** **und b** geben eine Handprothese mit drehbarer Anbindungskomponente **20,** Handflächenelement **19 (****Fig.4a****)** und mehreren Fingerelementen in der beschriebenen Ausführungsform aus zwei Perspektiven wieder. Sämtliche Fingerelemente sind über die Trägerkomponente an das Handflächenelement **19** befestigt. Zeige-, Mittel- und Ringfingerelement sind dabei starr an eine elastische Aufnahme **21 (****Fig.4b****)** angebunden, während für die Aufnahme des Daumenelements **24** eine separate Fingerelementaufnahme **22** mit einem zusätzlichen aktiv angetriebenen sowie elastisch um eine Mittelstellung federnden Gelenk vorgesehen sind. Auch das kleine Fingerelement **25** weist eine eigene elastisch nachgiebige Fingerelementaufnahme **23** auf.

Ein Daumenelement **24** mit der dazugehörigen Fingerelementaufnahme **22** ist zudem in **Fig.4a** **bis d** in verschiedenen Stellungen im Detail dargestellt. **Fig.4a** **und b** geben dabei Stellungen mit gestreckter Fingerelementaufnahme **22** wieder, während **Fig.4c** und d jeweils eine abgewinkelte Stellung der Fingerelementaufnahme zeigen. Die Fingerelementaufnahme weist hierzu eine motorisch angetriebene Gelenkverbindung auf, die vorzugsweise einer ersten Gelenkverbindung zwischen Trägerkomponente **1** und erstem Fingerglied **5** konzeptionell entspricht. Die Gelenkverbindung **22** in der dargestellten Ausführungsform umfasst folglich ein eigenes Antriebsgehäuse **26** (entsprechend dem ersten Fingerglied) mit einem motorischen Antrieb mit Gewindeschnecke und Zahnradsegment **27** um eine Schwenkachse **28** sowie eine Anbindungsplattform **29** für die Trägerkomponenten **1** des Daumenelements **25** (vgl. **Fig.4a****).**

Die Konzeption der Fingerelemente in den dargestellten Ausführungsformen lässt eine Anpassung an die jeweilige Verwendung allein durch einen Austausch der Fingerkuppe **18** und für einen Einsatz in einer Handprothese des Handflächenelement **19** sowie der elastischen Aufnahme **21** zu. Zudem sind die Fingerelementaufnahmen **22** und **25** gem. **Fig.3a** **und b** sowie **4a bis d** ausschließlich mit Komponenten des Fingerelements gefertigt. Das Antriebsgehäuse **26** ist mit allen Einbauteilen vorzugsweise baugleich mit dem ersten Fingerglied **5,** die Anbindungspattform **28** mit der Trägerkomponente **1.** Dies begünstigt eine Standardisierung der Komponenten oder ganzer Baugruppen, was wiederum eine wirtschaftliche Fertigung und Lagerhaltung sowie eine Anpassung auf individuelle Bedürfnisse des Prothesenträgers signifikant begünstigt.

Nicht in den Figuren dargestellt ist eine optische Verkleidung des Fingerelements oder der gesamten Handprothese z.B. mit einem Handschuh. Ein bevorzugter Handschuh aus einem elastomeren Material wie z.B. aus Silikonkautschuk hat gegenüber einem textilen oder ledernen Handschuh nicht nur optische Vorteile, sondern bietet einen besseren Schutz gegenüber Verschmutzungen.

### Bezugszeichenliste

| | |
|---|---|
| 1 | Trägerkomponente |
| 2 | erste Gelenkachse |
| 3 | Zahnradsegment |
| 4 | Zahnsegment |
| 5 | erstes Fingerglied |
| 6 | zweites Fingerglied |
| 7 | zweite Gelenkachse |
| 8 | Federstabverbindungen |
| 9 | Lagerbohrung an der Trägerkomponente |
| 10 | Lagerbohrung an zweitem Fingerglied |
| 11 | motorischer Antrieb |
| 12 | Antriebswelle |
| 13 | Gewindeschnecke |
| 14 | Führung |
| 15 | Kern |
| 16 | Abstreifer |
| 17 | Gleitfläche |
| 18 | Fingerkuppe |
| 19 | Handflächenelement |
| 20 | Anbindungskomponente |
| 21 | elastische Aufnahme |
| 22 | Fingerelementaufnahme für Daumenelement |
| 23 | Fingerelementaufnahme für kleine Fingerelement |
| 24 | Daumenelement |
| 25 | kleines Fingerelement |
| 26 | Antriebsgehäuse |
| 27 | Zahnradsegment |
| 28 | Schwenkachse |
| 29 | Anbindungsplattform |

## Patentansprüche

1. Fingerelement, umfassend
a) eine Trägerkomponente **(1),**
b) ein erstes Fingerglied **(5)** mit einer ersten Gelenkverbindung **(2)** zur Trägerkomponente,
c) ein zweites Fingerglied **(6)** mit einer zweiten Gelenkverbindung **(7)** zum ersten Fingerglied,
d) einen Stellantrieb für die. erste Gelenkverbindung **(2)** mit Motor **(11)** mit Antriebswelle **(12)** und Schneckengetriebe mit einer Gewindeschnecke **(13)** und einem auf die Gewindeschnecke eingreifenden Zahnsegment **(4)** sowie
e) einen Kopplungsmechanismus **(8)** zwischen ersten und zweiten Gelenkverbindung,
**dadurch gekennzeichnet, dass**
f) die Gewindeschnecke auf der Antriebswelle axial bewegbar formschlüssig gelagert sowie axial durch separate Führungen **(14)** geführt ist.

2. Fingerelement nach Anspruch 1, **dadurch gekennzeichnet, dass** der Motor und die Gewindeschnecke **(13)** sowie die Führungen **(14)** im ersten Fingerglied **(5)** angeordnet sind und das Zahnsegment **(4)** mit der Trägerkomponente **(1)** verbunden ist.

3. Fingerelement nach Anspruch 1 oder 2, **dadurch gekennzeichnet, dass** das Zahnsegment **(4)** durch ein Zahnradsegment **(3)** mit einer Zahnradachse gebildet wird, wobei die Zahnradachse eine Schwenkrichtung der ersten Gelenkverbindung vorgibt.

4. Fingerelement nach einem der vorgenannten Ansprüche, **dadurch gekennzeichnet, dass** die Führungen **(14)** und die Gewindeschnecke **(13)** zu einer Baugruppe zusammengefasst im ersten Fingerglied **(5)** eingesetzt ist.

5. Fingerelement nach Anspruch 4, **dadurch gekennzeichnet, dass** die Baugruppe elastisch eingesetzt ist.

6. Fingerelement nach einem der vorgenannten Ansprüche, **dadurch gekennzeichnet, dass** die Gewindeschnecke **(13)** radial zum Zahnsegment **(4)** in den Führungen **(14)** gelagert ist.

7. Fingerelement nach einem der vorgenannten Ansprüche, **dadurch gekennzeichnet, dass** der Kopplungsmechanismus mindestens eine elastische Verbindung **(8)** zwischen Trägerkomponente **(1)** und zweitem Fingerglied **(6)** parallel geschaltet zum ersten Fingerglied **(5)** aufweist.

8. Fingerelement nach Anspruch 7, **dadurch gekennzeichnet, dass** die Verbindung eine oder zwei Federstabverbindungen **(8)** als Zug-Druckstangen umfasst.

9. Fingerelement nach einem der vorgenannten Ansprüche, **dadurch gekennzeichnet, dass** das das zweite Fingerglied eine austauschbare Fingerkuppe **(18)** aufweist.

10. Fingerelement nach einem der vorgenannten Ansprüche, **dadurch gekennzeichnet, dass** das erste und/oder zweite Fingerglied einen taktilen Sensor oder einen Dehnungsmessstreifen zur Erfassung taktiler Signale umfasst.

11. Fingerelement nach Anspruch 8, **dadurch gekennzeichnet, dass** die Federstabverbindungen gebogen oder geknickt sind.

## Claims

1. Finger element, comprising
a) a carrier component (1),
b) a first phalanx (5) having a first articulated connection (2) to the carrier component,
c) a second phalanx (6) having a second articulated connection (7) to the first phalanx,
d) an actuator for the first articulated connection (2) with a motor (11) having a drive shaft (12) and a worm gear having a threaded worm (13) and a toothed segment engaging on the threaded worm,(4) and further comprising
e) a coupling mechanism (8) between the first and second articulated connections,
wherein
f) the threaded screw is supported on the drive shaft form-fittingly and axially movable as well as guided in axial direction by separate guidances (14).

2. A finger element according to claim 1, wherein the motor and the threaded screw (13) as well as the guidances (14) are arranged in the first phalanx (5) and the toothed segment (4) is connected to the carrier component (1).

3. A finger element according to claim 1 or 2, wherein the toothed segment (4) is formed by a gear segment (3) with a gear axis, whereby the gear axis predetermines a rotation axis of the first hinge connection.

4. A finger element according to one of the claims above, wherein, the guidances (14) and the threaded screw (13) are combined to an assembly that is inserted in the first phalanx(5).

5. A finger element according to claim 4, wherein the assembly is inserted elastically.

6. A finger element according to one of the claims above
wherein the threaded screw (13) is supported in the radial direction to the toothed segment (4) in the guidances (14).

7. A finger element according to one of the claims above
wherein the coupling mechanism comprises at least an elastic connection (8) between the carrier component (1) and the second phalanx (6) in parallel to the first phalanx (5).

8. A finger element according to claim 7 wherein the connection encloses one or two spring bar connections (8) as push-push rods.

9. A finger element according to one of the claims above,
wherein the second phalanx comprises an exchangeable finger tip (18).

10. A finger element according to one of the claims above,
wherein the first and/or second phalanx encloses a tactile sensor or a resistance strain gauge for detecting tactile signals.

11. A finger element according to claim 8, wherein the spring bar connections are formed in a curved or buckled manner.

## Revendications

1. Elément formant un doigt, comprenant
a) un élément de support (1),
b) une première section du doigt (5) avec un premier joint articulé (2) pour l'élément de support,
c) une deuxième section du doigt (6) avec un deuxième joint articulé (7) pour la première section du doigt,
d) un servomoteur pour le premier joint articulé (2), avec moteur (11) doté d'un arbre d'entraînement (12) et d'un engrenage à vis sans fin avec une vis sans fin taraudée (13) et un segment denté (4) s'engrenant sur la vis sans fin taraudée et
e) un mécanisme de couplage (8) entre le premier et le deuxième joint articulé, **caractérisé par le fait que**
f) la vis sans fin taraudée, axialement mobile, logée par emboîtement sur l'arbre d'entraînement et guidée axialement par des guides séparés (14).

2. Elément formant un doigt conformément à la revendication n°1, **caractérisé par le fait que** le moteur et la vis sans fin taraudée (13) ainsi que les guides (14) sont disposés dans la première section du doigt (5) et que le segment denté (4) est relié à l'élément de support (1).

3. Elément formant un doigt conformément à la revendication n°1 ou n°2, **caractérisé par le fait que** le segment denté (4) est formé d'un segment d'engrenage (3) doté d'un axe d'engrenage, ce dernier définissant le sens de pivotement du premier joint articulé.

4. Elément formant un doigt conformément à l'une des revendications susmentionnées, **caractérisé par le fait que** les guides (14) et la vis sans fin taraudée (13), réunis dans un module, sont mis en place dans la première section du doigt (5).

5. Elément formant un doigt conformément à la revendication n°4, **caractérisé par le fait que** le module est introduit élastiquement.

6. Elément formant un doigt conformément à l'une des revendications susmentionnées, **caractérisé par le fait que** la vis sans fin taraudée (13) est logée radialement par rapport au segment denté (4) dans les guides (14).

7. Elément formant un doigt conformément à l'une des revendications susmentionnées, **caractérisé par le fait que** le mécanisme de couplage présente au moins un raccord élastique (8) entre l'élément de support (1) et la deuxième section du doigt (6), montée en parallèle par rapport à la première section du doigt (5).

8. Elément formant un doigt conformément à la revendication n°7, **caractérisé par le fait que** l'assemblage comprend un ou deux raccords de tiges à ressorts (8) comme barres de traction-compression.

9. Elément formant un doigt conformément à l'une des revendications susmentionnées, **caractérisé par le fait que** la deuxième section du doigt présente un bout de doigt (18) échangeable.

10. Elément formant un doigt conformément à l'une des revendications susmentionnées, **caractérisé par le fait que** la première et/ou la deuxième section du doigt comprend un capteur tactile ou une jauge extensométrique pour l'enregistrement de signaux tactiles.

11. Elément formant un doigt conformément à la revendication n°8, **caractérisé par le fait que** les raccords de tiges à ressorts sont courbés ou pliés.
